# EUROPÄISCHE PATENTSCHRIFT

(11) **EP 3 161 468 B1**
(45) Veröffentlichungstag und Bekanntmachung des Hinweises auf die Patenterteilung: **15.04.2020**
(21) Anmeldenummer: 15730480.9
(22) Anmeldetag: 17.06.2015
(51) Int. Cl.: G01N 27/22, G01N 33/38

(54) **MESSVORRICHTUNG, INSBESONDERE FEUCHTIGKEITSMESSVORRICHTUNG**
MEASURING DEVICE, ESPECIALLY MOISTURE MEASURING DEVICE
DISPOSITIF DE MESURE, EN PARTICULIER DISPOSITIF DE MESURE D'HUMIDITÉ

(30) Priorität: 25.06.2014 DE 102014212136
(43) Veröffentlichungstag der Anmeldung: 03.05.2017
(73) Patentinhaber: Robert Bosch GmbH, 70442 Stuttgart (DE)
(72) Erfinder: KRAPF, Reiner, 70794 Filderstadt (DE); FRANK, Michael, 75015 Bretten (DE); BAIERL, Wolfgang, 73614 Schorndorf (DE)
(86) Internationale Anmeldenummer: PCT/EP2015/063595
(87) Internationale Veröffentlichungsnummer: WO 2015/197445

(56) Entgegenhaltungen:
- DE-A1-102012 208 050
- US-A1- 2006 117 833
- US-B1- 6 340 892
- US-B1- 7 038 470
- XIAOHUA JIN ET AL: "Reflection and transmission properties of embedded dipoles and PIFAs inside Concrete at 915 MHz", ANTENNAS AND PROPAGATION SOCIETY INTERNATIONAL SYMPOSIUM, 2009. APSURSI '09. IEEE, IEEE, PISCATAWAY, NJ, USA, Juni 2009 (2009-06), Seiten 1-4, XP031536137, ISBN: 978-1-4244-3647-7

## Beschreibung

### Stand der Technik

Es ist bereits eine Messvorrichtung, mit einer integrierten Sensoreinheit vorgeschlagen worden, welche dazu vorgesehen ist, eine Absolut- und/oder Relativfeuchte eines Feststoffes, insbesondere eines Baustoffs, zu bestimmen, beispielsweise in DE 10 2012 208 050 A1. Ferner wurden in der Publikation von Xiaohua Jin et al. "Reflection and transmission properties of embedded dipoles and PIFAs inside concrete at 915 MHz", IEEE, 2009 Sensoreinheiten zur Einbringung in einen Baustoff untersucht. Eine mobile Messvorrichtung zum Messen eines Feuchtigkeitskennwerts eines Baustoffs ist zudem aus der US 6340892 B1 bekannt.

### Offenbarung der Erfindung

Die Erfindung geht aus von einer Messvorrichtung, insbesondere einer Feuchtigkeitsmessvorrichtung, mit einer integrierten Sensoreinheit, welche dazu eingerichtet ist, zumindest einen Feuchtigkeitskennwert zumindest eines Baustoffs zu erfassen.

Es wird vorgeschlagen, dass die Messvorrichtung eine Kommunikationseinheit umfasst, welche wenigstens dazu eingerichtet ist, zumindest ein Signal zumindest einer externen Sensoreinheit zu empfangen.

Unter einer "Messvorrichtung" soll in diesem Zusammenhang eine als ein mobiles Gerät, vorzugsweise als ein Handgerät, ausgebildete Vorrichtung gemäß Anspruch 1 verstanden werden. Unter einer "Sensoreinheit" soll in diesem Zusammenhang insbesondere eine Einheit verstanden werden, welche dazu eingerichtet ist, physikalische und/oder chemische Eigenschaften und/oder die stoffliche Beschaffenheit ihrer Umgebung qualitativ und/oder als Messgröße quantitativ zu erfassen.

Unter einer "integrierten Sensoreinheit" soll in diesem Zusammenhang eine Sensoreinheit verstanden werden, welche zumindest teilweise innerhalb eines Gehäuses der Messvorrichtung angeordnet und/oder untrennbar mit weiteren Einheiten der Messvorrichtung, insbesondere mit der Kommunikationseinheit, verbunden ist. Die integrierte Sensoreinheit ist als eine Feuchtigkeitssensoreinheit ausgebildet, welche dazu eingerichtet ist, vorzugsweise zerstörungsfrei zumindest einen Feuchtigkeitskennwert eines Baustoffs zu erfassen. Insbesondere kann die integrierte Sensoreinheit den zumindest einen Feuchtigkeitskennwert radiometrisch und/oder resistiv und/oder kapazitiv und/oder mittels eines Mikrowellen-/Radarverfahrens und/oder mittels eines Aufsatz-NMR-Verfahrens erfassen.

Unter einem "Feuchtigkeitskennwert" soll in diesem Zusammenhang ein Kennwert verstanden werden, welcher zumindest eine Information über einen absoluten und/oder relativen Feuchtegehalt zumindest eines Baustoffs enthält. Unter einem "Baustoff" soll in diesem Zusammenhang ein Feststoff, insbesondere in Form eines Rohstoffs und/oder eines Hilfsstoffs und/oder eines Halbzeugs verstanden werden, welcher zu einer Verwendung bei einer Errichtung eines Bauwerks und/oder eines Gebäudes und/oder eines Bauteils vorgesehen ist. Unter einem "Bauteil" soll in diesem Zusammenhang ein einzelnes Teil, ein Element und/oder eine Komponente verstanden werden, aus welchem ein Bauwerk und/oder ein Gebäude zusammengesetzt ist. Bei einem Bauteil handelt es sich um eine/einen geometrisch zusammenhängende Wand- und/oder Bodenfläche und/oder Körper, die und/oder der einen/eine zumindest im Wesentlichen einheitlichen Aufbau und/oder Konstruktion aufweist. Unter einer "Kommunikationseinheit" soll in diesem Zusammenhang eine Einheit verstanden werden, welche dazu vorgesehen ist, zumindest ein analoges und/oder digitales Signal zumindest einer externen Sensoreinheit drahtgebunden und/oder vorzugsweise drahtlos zu empfangen und/oder zumindest ein Signal, insbesondere ein Steuer- und/oder Parametrisierungssignal, an die zumindest eine externe Sensoreinheit zu senden. Unter einem "Signal" soll in diesem Zusammenhang eine messbare physikalische Größe, insbesondere eine elektrische Spannung und/oder vorzugsweise ein elektrisches und/oder magnetisches Feld, verstanden werden, welcher zumindest eine Information zugeordnet und/oder aufgeprägt ist.

Unter einer "externen Sensoreinheit" soll in diesem Zusammenhang eine Sensoreinheit verstanden werden, welche dazu vorgesehen ist, abgesetzt von der Messvorrichtung betrieben zu werden. Die zumindest eine externe Sensoreinheit ist dazu eingerichtet zumindest einen weiteren Feuchtigkeitskennwert des zumindest einen Baustoffs unmittelbar in einem Inneren des zumindest einen Baustoffs zu erfassen und ihn drahtgebunden und/oder vorzugsweise drahtlos an die Kommunikationseinheit der Messvorrichtung zu übertragen.

Durch die erfindungsgemäße Ausgestaltung kann eine gattungsgemäße Messvorrichtung mit vorteilhaften Betriebseigenschaften bereitgestellt werden. Insbesondere können Kennwerte der integrierten Sensoreinheit und zumindest einer externen Sensoreinheit vorteilhaft parallel und/oder zeitversetzt erfasst werden. Hierdurch können vorteilhaft einfach und/oder schnell eine Vielzahl verschiedenartiger und/oder vorzugsweise gleichartiger Kennwerten erfasst und/oder ausgewertet werden.

Des Weiteren wird vorgeschlagen, dass die Kommunikationseinheit dazu vorgesehen ist, das zumindest eine Signal drahtlos zu empfangen und/oder zumindest ein Signal drahtlos zu senden. Unter der Wendung "drahtlos empfangen" soll verstanden werden, dass die Kommunikationseinheit dazu vorgesehen ist, das Signal über einen vorteilhaft körperlosen Informationsträger, beispielsweise über Schall-, Licht- und/oder vorzugsweise Funkwellen, zu empfangen.

Vorzugsweise ist die Kommunikationseinheit als eine RFID-Kommunikationseinheit (RFID = Radio Frequency Identification) ausgebildet. Hierdurch kann eine vorteilhaft komfortable Übermittlung des zumindest einen Signals von der zumindest einen externen Sensoreinheit an die Messvorrichtung ermöglicht werden. Ferner kann die Kommunikationseinheit vorteilhaft einfach zu einem drahtlosen Senden von Signalen verwendet werden.

Erfindungsgemäß wird vorgeschlagen, dass die Messvorrichtung eine Recheneinheit umfasst, welche dazu eingerichtet ist, den zumindest einen Feuchtigkeitskennwert mit dem zumindest einen weiteren Feuchtigkeitskennwert zu verrechnen. Unter einer "Recheneinheit" soll insbesondere eine Einheit verstanden werden, die von einer Auswerteeinheit und/oder einer Kontrolleinheit gebildet sein kann, wobei die Recheneinheit sowohl von einem Prozessor allein als auch insbesondere von einem Prozessor und weiteren Elektronikbauteilen, wie beispielsweise einem Speichermittel, gebildet sein kann. Darunter, dass der zumindest eine Feuchtigkeitskennwert mit dem zumindest einen weiteren Feuchtigkeitskennwert "verrechnet" wird, soll insbesondere verstanden werden, dass der zumindest eine Feuchtigkeitswert und der zumindest eine weitere Feuchtigkeitskennwert insbesondere als Parameter in zumindest eine von der Recheneinheit ausgeführte mathematische und/oder logische Operation einfließen. Ein Ergebnis der zumindest einen mathematischen und/oder logischen Operation kann insbesondere zu einer Weiterverwendung gespeichert und/oder zwischengespeichert und/oder an einen Benutzer ausgegeben werden. Hierdurch wird eine vorteilhafte Auswertung der Feuchtigkeitskennwerte ermöglicht. Insbesondere können der zumindest eine Feuchtigkeitswert und der zumindest eine weitere Feuchtigkeitswert vorteilhaft zueinander in Relation gesetzt werden.

Ferner wird vorgeschlagen, dass die Recheneinheit dazu eingerichtet ist, aus dem zumindest einen Feuchtigkeitskennwert und dem zumindest einen weiteren Feuchtigkeitskennwert zumindest eine Kompensationskenngröße zu ermitteln. Unter einer "Kompensationskenngröße" soll in diesem Zusammenhang insbesondere eine Kenngröße verstanden werden, welche zumindest eine, insbesondere messtechnisch bedingte, Abweichung zwischen dem zumindest einen Feuchtigkeitskennwert und dem zumindest einen weiteren Feuchtigkeitskennwert widerspiegelt. Insbesondere ist durch eine Anwendung der zumindest einen Kompensationsgröße auf den zumindest einen Feuchtigkeitskennwert eine Abweichung des zumindest einen Feuchtigkeitskennwerts gegenüber dem zumindest einen weiteren Feuchtigkeitskennwert zumindest im Wesentlichen kompensierbar.

Hierdurch können vorteilhaft einfache und/oder präzise insbesondere auf Grund zueinander verschiedenere Messverfahren auftretende Abweichungen zwischen dem zumindest einen Feuchtigkeitskennwert und dem zumindest einen weiteren Feuchtigkeitskennwert zumindest weitgehend korrigiert werden. Insbesondere können Messfehler der internen Sensoreinheit somit vorteilhaft kompensiert werden.

Weist die Messvorrichtung zumindest eine Schnittstelle auf, welche dazu vorgesehen ist, Daten an zumindest eine externe Datenverarbeitungseinheit zu übermitteln, können mittels der Messvorrichtung erfasste und/oder erzeugte und/oder zwischengespeicherte Daten vorteilhaft einfach insbesondere zu einer Speicherung und/oder Weiterverarbeitung von der Messvorrichtung abgerufen werden. Unter einer "Schnittstelle" soll in diesem Zusammenhang insbesondere eine Datenschnittstelle verstanden werden, über welche die Messvorrichtung drahtlos und/oder drahtgebunden mit zumindest einer externen Datenverarbeitungseinheit koppelbar ist. Unter einer "externen Datenverarbeitungseinheit" soll in diesem Zusammenhang insbesondere eine von der Messvorrichtung unabhängige Einheit verstanden werden, welche insbesondere dazu vorgesehen ist, Daten aufzunehmen und/oder zu speichern und/oder aufzubereiten und/oder auszuwerten.

Vorzugsweise umfasst die Messvorrichtung eine Energiesendeeinheit, welche zu einer kontaktlosen Energieversorgung der zumindest einen externen Sensoreinheit eingerichtet ist. Unter einer "Energiesendeeinheit" soll in diesem Zusammenhang insbesondere eine Einheit verstanden werden, welche dazu vorgesehen ist, insbesondere eine elektrische Energie mittels eines induktiven und/oder elektromagnetischen Verfahrens an die zumindest eine externe Sensoreinheit zu übertragen. Vorzugsweise ist die Energiesendeeinheit einstückig mit der Kommunikationseinheit ausgebildet. Hierdurch kann die zumindest eine externe Sensoreinheit vorteilhaft einfach mit elektrischer Energie versorgt werden. Insbesondere kann auf eine Energiequelle innerhalb der zumindest einen externen Sensoreinheit verzichtet werden, wodurch eine Laufzeit der zumindest einen externen Sensoreinheit vorteilhaft verlängerbar ist.

Vorzugsweise ist die Energiesendeeinheit einstückig mit der Kommunikationseinheit ausgebildet. Das bedeutet, dass die Energiesendeeinheit und die Kommunikationseinheit durch dieselbe Einheit bzw. dieselbe Baugruppe gebildet sind. Die Energiesendeeinheit dient in vorteilhafter Weise gleichzeitig auch als Kommunikationseinheit. Hierdurch kann die zumindest eine externe Sensoreinheit vorteilhaft einfach mit elektrischer Energie versorgt werden. Insbesondere kann auf eine Energiequelle innerhalb der zumindest einen externen Sensoreinheit verzichtet werden, wodurch eine Laufzeit der zumindest einen externen Sensoreinheit vorteilhaft verlängerbar ist.

Ferner wird eine externe Sensoreinheit zur Verwendung mit einer sensorbehafteten Messvorrichtung nach Anspruch 1 vorgeschlagen, wobei die externe Sensoreinheit zumindest einen Sensor aufweist, welcher dazu vorgesehen ist, einen Feuchtigkeitskennwert zumindest eines Baustoffs zu erfassen. Der Sensor kann dabei ein kapazitiver Feuchtesensor oder auch ein wasserdichter MEMS-Luftfeuchtesensor sein. Der Sensor der externen Sensoreinheit ist dabei insbesondere ein die Luftfeuchte messender Sensor, der über eine Membran die Ausgleichsfeuchte mit dem Baustoff messen kann. Alternativ oder zusätzlich kann ein Feuchtesensor, der über Elektroden entweder kapazitiv oder resistiv mit dem Baustoff in Berührung steht und so die lokale Feuchte bestimmt, Verwendung finden.

Vorteilhafterweise kann die als insbesondere wasserdichtes Modul ausgestaltete externe Sensorvorrichtung die Temperatur im Baustoff messen, durch welche dann der Taupunkt im Mauerwerk bestimmbar wird. Dazu kann die externe Sensorvorrichtung zusätzlich auch zumindest einen Temperatursensor aufweisen.

Die Messdaten der externe Sensoreinheit, wie beispielsweise Temperatur und Feuchte können in vorteilhafter Weise mittels der Messvorrichtung nach Anspruch 1, die von der externen Sensoreinheit getrennt ist, von dieser aber vorzugsweise mit Energie versorgt wird, ausgelesen werden.

Vorzugsweise umfasst die externe Sensoreinheit eine Energiebereitstellungseinheit, welche zu einer kontaktlosen Energieversorgung der zumindest einen externen Sensoreinheit, insbesondere der Sensoren oder des Sensors der externen Sensoreinheit, vorgesehen ist. Unter einer "Energiebereitstellungseinheit" soll in diesem Zusammenhang insbesondere eine Einheit verstanden werden, welche dazu vorgesehen ist, insbesondere eine elektrische Energie mittels eines induktiven und/oder elektromagnetischen Verfahrens anzunehmen und den Komponenten der externe Sensoreinheit zur Verfügung zu stellen.

Die Energiebereitstellungseinheit kann beispielsweise ein RFID-Tag oder induktives System, optional mit Übergangsenergiespeicher, wie beispielsweise einem Akku oder einem Kondensator sein, und Energie eines externen Senders, wie beispielsweise der Energiesendeeinheit der Messvorrichtung erhalten.

Zudem umfasst die externe Sensoreinheit eine Datenkommunikationseinheit, wie beispielsweise ein RFID-Tag oder ein Bluetooth low-energy Modul, ein ZigBee Modul o.ä. Optional kann ein Messwertspeicher und/oder eine Ablaufregelung, insbesondere für den Sensor, die dazu vorgesehen ist, Daten der externen Sensoreinheit, wie beispielsweise einen Feuchtigkeitskennwert, an die zugeordnete, von der externen Sensoreinheit aber getrennte Messvorrichtung nach Anspruch 1, insbesondere eine handgehaltene Messvorrichtung, weiterzugeben. Vorzugsweise ist die Kommunikationseinheit dazu vorgesehen mit der Kommunikationseinheit der Messvorrichtung insbesondere bidirektional zu kommunizieren.

Vorzugsweise sind die Energiebereitstellungseinheit und die Datenkommunikationseinheit der externen Sensorvorrichtung durch dieselbe Komponente gebildet. Diese Einheit (z.B. ein RFID-Tag) übernimmt die Energieversorgung des externen Feuchtesensors und die Datenübertragung nach außen, aus dem Baustoff heraus.

Vorzugsweise wird der Feuchtesensor der externen Sensoreinheit dabei aber nicht von der Datenkommunikationseinheit, also beispielsweise von einem RFID-Tag, gebildet, sondern ist als separates Bauteil, wie beispielsweise ein kapazitiver Feuchtesensor oder ein MEMS-Luftfeuchtesensor (MEMS = Microelectromechanical System) ausgebildet und wird über die Datenkommunikationseinheit lediglich ausgelesen und auch mit Energie versorgt.

Vorzugsweise kann die Energiebereitstellungseinheit der externen Sensoreinheit auch durch das Aufladen eines Übergangsenergiespeichers, wie beispielsweise in der externen Sensoreinheit vorgesehenen Akkus oder Kondensatoren, eine Zeitlang autark, d.h. ohne aktive Energieversorgung von Außen, Sensorwerte aufnehmen und speichern.

In alternativen Ausführungsformen kann die externe Sensoreinheit auch intrinsisch Energie erzeugen, beispielsweise über Gebäudevibrationen oder Osmosepotentiale feuchter Baustoffe.

Auch kann die externe Sensoreinheit ein elektrochemisches Element bilden und über die vorhandene Feuchte im Material als galvanische Zelle arbeiten.

Die externe Sensoreinheit hat in vorteilhafter Weise eine bekannte, einzigartige Kennung einprogrammiert, so dass der Einbauort mit Hilfe einer "elektronischen Landkarte" mittels eines Lesegerätes, wie beispielsweise der handgehaltenen Messvorrichtung, und den Informationen eines Bedieners einmalig festgelegt wird.

Der in der externen Sensoreinheit vorhandene Feuchtesensor wird dazu ausgelesen, seine Kennung notiert, der Einbauort im Lesegerät oder in einem Computer notiert, so dass sich eine Karte aller verbauten Sensoren eines Raumes oder Gebäudes ergibt. Im Nachgang kann ein Bediener einfach am Bauteil vorbeigehen, wobei die Daten automatisch ausgelesen und dem Einbauort zugeordnet werden können. Somit sind auch Langzeitmessungen möglich, und die Sensorposition kann auch mit einem Bauplan kombiniert werden oder dort verzeichnet sein.

Ferner wird ein Messsystem nach Anspruch 6, insbesondere ein Feuchtigkeitsmesssystem, mit zumindest einer Messvorrichtung und mit der zumindest einer externen Sensoreinheit vorgeschlagen, welche dazu eingerichtet ist, zumindest einen weiteren Feuchtigkeitskennwert des zumindest einen Baustoffs zu erfassen. Vorzugsweise umfasst das Feuchtigkeitsmesssystem eine Mehrzahl von externen Sensoreinheiten, welche vorzugsweise beabstandet zueinander an oder in dem zumindest einen Baustoff angeordnet sind. Hierdurch kann eine vorteilhafte Erfassung von Feuchtigkeitsmesswerten zumindest eines Baustoffs erreicht werden.

Ist die zumindest eine externe Sensoreinheit zu einer zumindest teilweisen und vorzugsweise vollständigen Einbettung in den zumindest einen Baustoff eingerichtet, kann eine vorteilhaft genaue Erfassung des zumindest einen weiteren Feuchtigkeitskennwerts erfolgen. Insbesondere können Messfehler durch ein von außerhalb des zumindest einen Baustoffs durchgeführten Messverfahrens weitgehend eliminiert werden.

Des Weiteren wird vorgeschlagen, dass die zumindest eine externe Sensoreinheit eine weitere Kommunikationseinheit aufweist, welche dazu eingerichtet ist, das zumindest eine Signal auszusenden. Vorzugsweise ist die eine weitere Kommunikationseinheit dazu vorgesehen mit der Kommunikationseinheit der Messvorrichtung insbesondere bidirektional zu kommunizieren.

Vorzugsweise ist die weitere Kommunikationseinheit als eine weitere RFID-Kommunikationseinheit ausgebildet. Hierdurch kann eine vorteilhafte Kommunikation zwischen der zumindest einen externen Sensoreinheit und der Messvorrichtung und insbesondere eine vorteilhafte Übertragung des zumindest einen weiteren Feuchtigkeitskennwerts von der zumindest einen externen Sensoreinheit an die Messvorrichtung erreicht werden.

Vorzugsweise weist die zumindest eine externe Sensoreinheit eine Energieempfangseinheit zu einer kontaktlosen Energieversorgung auf.

Unter einer "Energieempfangseinheit" soll in diesem Zusammenhang insbesondere eine Einheit verstanden werden, welche dazu vorgesehen ist, eine insbesondere von einer Energiesendeeinheit induktiv und/oder elektromagnetisch übertragene elektrische Energie aufzunehmen und/oder aufzubereiten. Hierdurch kann die zumindest eine externe Sensoreinheit vorteilhaft einfach mit elektrischer Energie versorgt werden. Vorzugsweise ist die Energieempfangseinheit einstückig mit der weiteren Energieversorgungseinheit ausgebildet. Insbesondere kann auf eine Energiequelle innerhalb der zumindest einen externen Sensoreinheit verzichtet werden, wodurch eine Laufzeit der zumindest einen externen Sensoreinheit vorteilhaft verlängerbar ist.

Eine vorteilhafte Ausführungsform des Feuchtigkeitsmesssystems weist zumindest eine, bevorzugt jedoch mehrere, externe Sensoreinheiten und eine als Lesegerät zum Ausgelesen der Daten der externe Sensoreinheit ausgebildete Messvorrichtung auf, welche zumindest eine Energiesendeeinheit, welche zu einer kontaktlosen Energieversorgung der zumindest einen externen Sensoreinheit vorgesehen ist, aufweist. Das Lesegerät selbst braucht dann nicht über einen aktiven Feuchtesensor zu verfügen, sondern kann lediglich der Feuchtigkeitskennwert der zugeordneten externen Sensoreinheiten auslesen.

Damit wird eine Vorrichtung vorgeschlagen, bei dem ein als externe Sensoreinheit ausgebildetes Modul, insbesondere ein wasserdichtes Modul, einen Feuchtesensor mit einer Kommunikationskomponente, wie beispielsweise einem RFID-Tag, kombiniert. In dem wasserdichten Modul, welches zum Verbleib in einem Baustoff vorgesehen, ist in vorteilhafter Weise ein Feuchtesensor mit geringer Leistungsaufnahme - beispielsweise ein kapazitiv arbeitender Feuchtesensor, der die Luftfeuchte misst und über eine Membran mit dem Baustoff die Ausgleichsfeuchte messen kann - oder ein Feuchtesensor, der über Elektroden entweder kapazitiv oder resistiv mit dem Baustoff in Berührung steht und so die lokale Feuchte bestimmen kann, integriert und mit einer Datenauslese- und Energiekomponente kombiniert. Das als externe Sensoreinheit ausgebildete Modul besitzt in vorteilhafter Weise eine Kommunikationskomponente, die sowohl zur Datenkommunikation als für das Energiemanagement des Feuchtesensors ausgebildet ist. Dabei bilden Feuchtesensor und Datenauslese- bzw. Energiekomponente unterschiedliche Bauelemente. Die Feuchtebestimmung erfolgt nicht über die Datenauslese- bzw. Energiekomponente, wie das aus dem Stand der Technik bekannt ist.

Vorteilhafterweise kann die als insbesondere wasserdichtes Modul ausgestaltete externe Sensorvorrichtung die Temperatur im Baustoff messen, durch welche dann der Taupunkt im Mauerwerk bestimmbar wird. Dazu weist die erfindungsgemäße externe Sensoreinheit zumindest auch einen Temperatursensor auf. So kann aufgrund der Materialfeuchte und Materialtemperatur ein Taupunkt im Material bestimmt werden. (Dies ist beispielsweise über das Glaser-Verfahren bzw. nach DIN 4108-3 möglich) Bei mehreren in den Baustoff eingebrachten Sensoren in verschiedenen Einbettungstiefen, kann in vorteilhafter Weise auch der Ort des aktuellen Taupunktes innerhalb des Baustoffes, beispielsweise innerhalb eines Mauerwerks, bestimmt und extern ausgelesen werden.

Dabei kann die Berechnung des Taupunktes durch die externe Sensoreinheit selbst vorgenommen werden und diese die Kennwerte für Feuchtigkeit, Temperatur und Taupunkt vorhalten, speichern, loggen und ggf. ausgeben. Alternativ kann ein externes Gerät, wie beispielsweise ein Auslesegerät oder auch die Messvorrichtung, die Temperatur und Luftfeuchte auslesen und daraus den Taupunkt berechnen.

Ebenfalls möglich ist es, die Materialtemperatur über die externe Sensoreinheit auszulesen und zusammen mit einem Messwert für die Luftfeuchte dann einen Taupunkt auf der Oberfläche des Baustoffes zu bestimmen. So kann dann mit einem externen Auslesegerät, welches die Sensordaten der externen Sensoreinheit ausliest - zusammen mit der ebenfalls gemessenen Luftfeuchtigkeit - ein Taupunkt auf der Bauwerksoberfläche bestimmt werden.

Bei einer Temperaturabhängigkeit des Feuchtesensors in der externen Sensoreinheit kann durch den gemessenen Temperaturwert in vorteilhafter Weise eine ggf. auftretende Abweichung/Drift des Feuchtesensors kompensiert werden.

Das Modul wird bei der Herstellung des Bauwerkes in den Baustoff mit eingebracht - beispielsweise einbetoniert - und kann dann von "außen" mit Energie versorgt und ausgelesen werden.

### Zeichnung

Weitere Vorteile ergeben sich aus der folgenden Zeichnungsbeschreibung. In der Zeichnung ist ein Ausführungsbeispiel der Erfindung dargestellt.

Es zeigt:

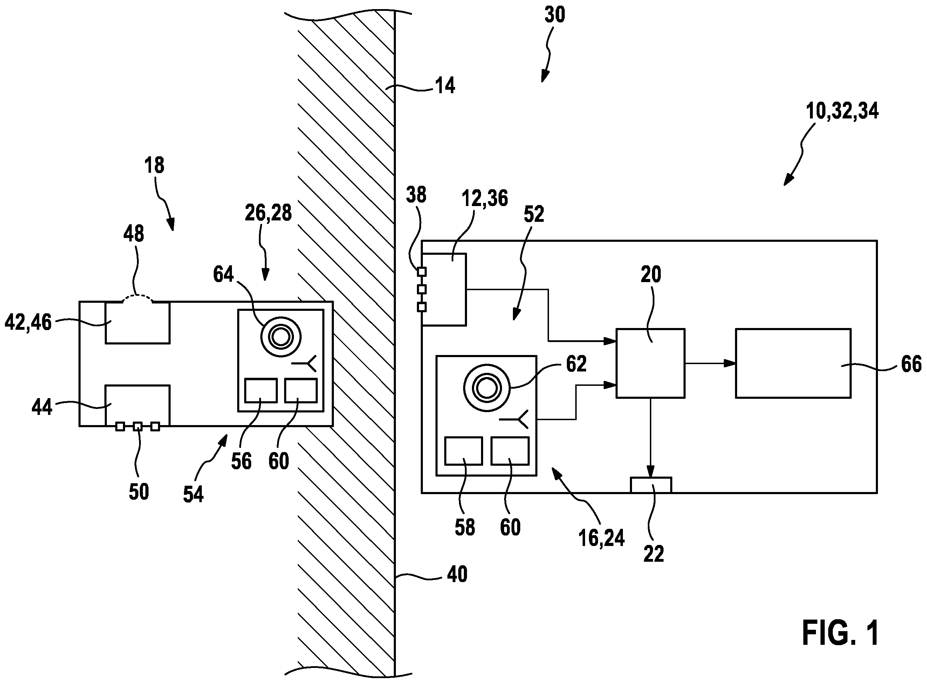

- Fig. 1: eine schematische Darstellung eines Feuchtigkeitsmesssystems mit einer Messvorrichtung, welche eine integrierte Sensoreinheit aufweist, und einer in einen Baustoff eingebetteten externen Sensoreinheit.

### Beschreibung des Ausführungsbeispiels

Figur 1 zeigt eine schematische Darstellung eines Feuchtigkeitsmesssystems 30 mit einer Messvorrichtung 10 und einer externen Sensoreinheit 18. Die Messvorrichtung 10 ist als eine Feuchtigkeitsmessvorrichtung 32 ausgebildet. Vorzugsweise ist die Messvorrichtung 10 als ein Handgerät 34 ausgebildet. Die Messvorrichtung 10 ist insbesondere dazu vorgesehen, eine Material- und/oder Baufeuchte eines Baustoffs 14, beispielweise einer Wand und/oder eines Bodens eines Gebäudes oder eines Bauwerks, zu bestimmen.

Hierzu weist die Messvorrichtung 10 eine integrierte Sensoreinheit 12 auf, welche dazu eingerichtet ist, einen Feuchtigkeitskennwert des Baustoffs 14 zu erfassen, mittels dessen auf einen Wassergehalt des Baustoffs 14 geschlossen werden kann. Die integrierte Sensoreinheit 12 ist hier beispielhaft als kapazitive Sensoreinheit 36 ausgebildet. Die Sensoreinheit 12 weist drei Elektroden 38 auf. Zur Erfassung des Feuchtigkeitskennwerts werden die Elektroden 38 der integrierten Sensoreinheit 12 in Kontakt mit einer Oberfläche 40 des Baustoffs 14 gebracht. Über eine Bestimmung einer Materialpermittivität mittels einer Wechselspannung zwischen den Elektroden 38 kann insbesondere bei bekannter Dielektrizitätskonstante des Baustoffs 14 in trockenem Zustand auf einen Wasseranteil innerhalb des Baustoffs 14 geschlossen werden. Alternativ und/oder zusätzlich ist es denkbar, dass eine Sensoreinheit zumindest eine Sensorik aufweist, welche dazu vorgesehen ist, einen Feuchtigkeitskennwert eines Baustoffs beispielsweise radiometrisch und/oder resistiv und/oder mittels eines Mikrowellen-/Radarverfahrens und/oder mittels eines Aufsatz-NMR-Verfahrens zu erfassen. Auf die genannten, dem Fachmann bekannten, Verfahren soll hier nicht im Detail eingegangen werden. Ferner ist die Aufzählung der Verfahren nicht als abschließend anzusehen, eine Verwendung weiterer, dem Fachmann als geeignet erscheinender Verfahren ist ebenso denkbar.

Besonders vorteilhaft ist es, wenn die externe Sensorvorrichtung 18 auch die Temperatur messen und gegebenenfalls loggen kann. Dazu kann die externe Sensorvorrichtung 18 in vorteilhaften Ausführungsformen zumindest einen Temperatursensor aufweisen.

So kann über die Materialfeuchte und die Materialtemperatur ein Taupunkt im Material 14 selbst bestimmt werden. Bei mehreren, in verschiedenen Einbettungstiefen in den Baustoff 14 eingebrachten, externen Sensorvorrichtungen 18, kann in vorteilhafter Weise auch der Ort des aktuellen Taupunktes innerhalb des Baustoffes 14, beispielsweise eines Mauerwerks, bestimmt werden.

Des Weiteren weist die Messvorrichtung 10 eine Kommunikationseinheit 16 auf, welche dazu reingerichtet ist, ein Signal einer externen Sensoreinheit 18 des Feuchtigkeitsmesssystems 30 zu empfangen. Die Kommunikationseinheit 16 ist vorzugsweise als eine RFID-Leseeinheit 52 ausgebildet.

Die externe Sensoreinheit 18 ist vollständig in den Baustoff 14 eingebettet, kann in anderen Anwendungen aber auch nur teilweise in den Baustoff 14 eingebettet sein. Die externe Sensoreinheit 18 umfasst hier beispielhaft zwei Sensoren 42, 44, welche jeweils dazu eingerichtet sind, einen Feuchtigkeitskennwert des Baustoffs 14 zu erfassen. Ebenso ist jedoch die Verwendung lediglich eines Sensors 42 oder 44 und/oder lediglich eines Sensortyps denkbar. Einer der Sensoren 42 ist als kapazitiver Feuchtigkeitssensor 46 zur Erfassung einer Luftfeuchtigkeit ausgebildet. Der Sensor 42 steht über eine luftdurchlässige jedoch wasserundurchlässige Membran 48 mit dem Baustoff 14 in Kontakt, wodurch eine Erfassung einer Ausgleichsfeuchte, welche Aufschluss auf einen Wassergehalt des Baustoffs 14 gibt, möglich ist. Der zweite Sensor 44 weist Elektroden 50 auf, welche mit dem Baustoff 14 in Kontakt stehen. Über die Elektroden 50 ist beispielsweise kapazitiv und/oder resistiv eine Materialpermittivität des Baustoffs 14, welche Aufschluss auf einen Wassergehalt des Baustoffs 14 gibt, erfassbar. Da die externe Sensoreinheit 18 unmittelbar in den Baustoff 14 eingebettet ist, kann ein Feuchtigkeitskennwert des Baustoffs 14 exakt erfasst und somit ein Wassergehalt des Baustoffs 14 präzise bestimmt werden. Zusätzlich kann die externe Sensoreinheit 18 einen hier nicht dargestellten Temperatursensor aufweisen. Mittels erfasster Temperaturwerte kann in Kombination mit dem Feuchtigkeitskennwert ein Taupunkt berechnet werden. Ferner kann eine eventuelle Temperaturdrift der Sensoren 42 kompensiert werden.

Zur Übertragung eines Signals, welches die durch die Sensoren 42, 44 erfassten Feuchtigkeitskennwerte als Nutzdaten beinhaltet, von der externen Sensoreinheit 18 an die Messvorrichtung 10, weist die externer Sensoreinheit 18 eine weitere Kommunikationseinheit 26 auf. Die weitere Kommunikationseinheit 26 ist vorzugsweise als ein insbesondere passiver RFID-Transponder 54 ausgebildet. Mittels der Kommunikationseinheit 26 der Messvorrichtung 10 sind von der externen Sensoreinheit 18 erfasste Feuchtigkeitskennwerte von der externen Sensoreinheit 18 drahtlos abrufbar und werden drahtlos von der weiteren Kommunikationseinheit 26 der externen Sensoreinheit 18 an die Kommunikationseinheit 16 der Messvorrichtung 10 übertragen.

Sowohl die Kommunikationseinheit 16 der Messvorrichtung 10 als auch die weitere Kommunikationseinheit 26 der externen Sensoreinheit 18 weisen eine Verarbeitungseinheit 56, 58 auf, welche beispielsweise zu einer Zwischenspeicherung von Daten und/oder einer Signalverarbeitung vorgesehen sind.

Ferner dient die Kommunikationseinheit 16 als eine Energiesendeeinheit 24, welche dazu vorgesehen ist, die externe Sensoreinheit 18 kontaktlos mit elektrischer Energie zu versorgen.

Eine Aufnahme der elektrischen Energie erfolgt durch die weitere Kommunikationseinheit 26 der externen Sensoreinheit 18, welche als Energieempfangseinheit 28 dient. Diese Komponente der externen Sensoreinheit 18 (z.B. ein RFID-Tag) übernimmt somit in vorteilhafter Weise sowohl die Energieversorgung des Feuchtesensors 42 bzw. 44 und die Datenübertragung der ermittelten Sensordaten nach außen zu der Messvorrichtung. Dadurch muss der Feuchtesensor 42 bzw. 44der externe Sensoreinheit 18 keine eigene Energieversorgung besitzen und kann direkt im Innern der Bausubstanz zerstörungsfrei messen, da er drahtlos ausgelesen werden kann.

Die Energiesendeeinheit 24 als auch die Energieempfangseinheit 28 weisen einen Pufferspeicher 60, beispielsweise einen Kondensator, zu einer kurzzeitigen Zwischenspeicherung elektrischer Energie auf. Sowohl eine Signalübertragung als auch eine Energieübertragung erfolgt über eine Antennen 62, 64 der Kommunikationseinheiten 16, 26.

Die von der integrierten Sensoreinheit 12 der Messvorrichtung 10 empfangenen Feuchtigkeitskennwerte und die von der Kommunikationseinheit 16 der Messvorrichtung 10 empfangenen weiteren, von der externen Sensoreinheit 18 erfassten, Feuchtigkeitskennwerte werden durch eine Recheneinheit 20 der Messvorrichtung 10 miteinander verrechnet. Bei dieser Verrechnung wird eine Kompensationskenngröße ermittelt, welche eine Abweichung zwischen den exakten Feuchtigkeitskennwerten der externen Sensoreinheit 18 und den messungenauigkeitsbehafteten Feuchtigkeitskennwerten der integrierten Sensoreinheit 12 widerspiegelt.

Mit Hilfe der ermittelten Kompensationskenngröße ist eine Messungenauigkeit der integrierten Sensoreinheit 12 zuverlässig kompensierbar.

Zur Informationsausgabe an einen Bediener weist die Messvorrichtung 10 eine Anzeigeeinheit 66 auf, über welche Informationen über einen Wassergehalt des Baustoffs 14 ausgebbar sind. Ferner weist die Messvorrichtung 10 eine Schnittstelle 22 auf, über welche Daten drahtlos und/oder drahtgebunden von der Messvorrichtung 10 auf externe hier nicht dargestellte, Datenverarbeitungsgeräte, beispielsweise PCs, Laptops und/oder Tablet-PCs, übertragbar sind.

Weist das Feuchtigkeitsmesssystem 30 eine Mehrzahl von externen Sensoreinheiten 18 auf, welche verteilt in den Baustoff 14 eingebettet sind, so können diese Mittels der Messvorrichtung 10 ausgelesen werden, wodurch exakte Feuchtigkeitskennwerte des Baustoffs 14 erfasst werden können. Bei einer ausreichenden Abdeckung des Baustoffs 14 mit einer Mehrzahl von externen Sensoreinheiten 18 kann in diesem Fall auf eine Verwendung der integrierten Sensoreinheit 12 verzichtet werden.

Weist das Feuchtigkeitsmesssystem 30 lediglich eine externe Sensoreinheit 18 auf, kann diese mittels der Messvorrichtung 10 ausgelesen werden, um einen exakten Feuchtigkeitskennwert für den Baustoff 14 am Einbauort der externen Sensoreinheit 18 zu erhalten. Mittels der integrierten Sensoreinheit 12 erfolgt daraufhin ebenfalls eine Erfassung eines Feuchtigkeitskennwerts an einer Oberfläche 40 des Baustoffs 14 im Bereich der externen Sensoreinheit 18. Durch die Recheneinheit 20 wird anhand der erfassten Feuchtigkeitskennwerte eine Kompensationskenngröße ermittelt. Bei gleichbleibendem Baustoff 14 können nun Feuchtigkeitskennwerte, welche mittels der integrierten Sensoreinheit 12 an anderer Stelle erfasst werden, mittels der Kompensationskenngröße korrigiert werden. Dies erlaubt eine exakte Erfassung von Feuchtigkeitskennwerten mittels der integrierten Sensoreinheit 12.

## Patentansprüche

1. Mobile Messvorrichtung, insbesondere Feuchtigkeitsmessvorrichtung, mit einer integrierten Sensoreinheit (12), die dazu eingerichtet ist, zumindest einen Feuchtigkeitskennwert zumindest eines Baustoffs (14) zu erfassen, und mit einer Kommunikationseinheit (16), welche wenigstens dazu eingerichtet ist, zumindest ein Signal zumindest einer externen Sensoreinheit (18) zu empfangen, wobei das zumindest eine Signal zumindest einen weiteren Feuchtigkeitskennwert des zumindest einen Baustoffs (14) beinhaltet, **gekennzeichnet durch** eine Recheneinheit (20), welche dazu eingerichtet ist, den zumindest einen Feuchtigkeitskennwert mit dem zumindest einen weiteren Feuchtigkeitskennwert zu verrechnen.

2. Mobile Messvorrichtung nach Anspruch 1, **dadurch gekennzeichnet, dass** die Kommunikationseinheit (16) dazu eingerichtet ist, das zumindest eine Signal drahtlos zu empfangen.

3. Mobile Messvorrichtung nach Anspruch 1, **dadurch gekennzeichnet, dass** die Recheneinheit (20) dazu eingerichtet ist, aus dem zumindest einen Feuchtigkeitskennwert und dem zumindest einen weiteren Feuchtigkeitskennwert zumindest eine Kompensationskenngröße zu ermitteln.

4. Mobile Messvorrichtung nach einem der vorhergehenden Ansprüche, **gekennzeichnet durch** zumindest eine Schnittstelle (22), welche dazu eingerichtet ist, Daten an zumindest eine externe Datenverarbeitungseinheit zu übermitteln.

5. Mobile Messvorrichtung nach einem der vorhergehenden Ansprüche, **gekennzeichnet durch** eine Energiesendeeinheit (24), welche zu einer kontaktlosen Energieversorgung der zumindest einen externen Sensoreinheit (18) eingerichtet ist.

6. Feuchtigkeitsmesssystem mit zumindest einer mobilen Messvorrichtung (10) nach einem der Ansprüche 1 bis 5 und zumindest einer externen Sensoreinheit, wobei die externe Sensoreinheit (18) zumindest einen Sensor (42,44) aufweist, welcher dazu eingerichtet ist, zumindest einen Feuchtigkeitskennwert eines Baustoffs (14) zu erfassen.

7. Feuchtigkeitsmesssystem nach Anspruch 6, **dadurch gekennzeichnet, dass** die zumindest eine externe Sensoreinheit (18) zu einer zumindest teilweisen Einbettung in den zumindest einen Baustoff (14) eingerichtet ist.

8. Feuchtigkeitsmesssystem nach Anspruch 6 oder 7, **dadurch gekennzeichnet, dass** die zumindest eine externe Sensoreinheit (18) eine weitere Kommunikationseinheit (26) aufweist, welche dazu eingerichtet ist, das zumindest eine Signal auszusenden.

9. Feuchtigkeitsmesssystem nach einem der Ansprüche 6 bis 8, **dadurch gekennzeichnet, dass** die zumindest eine externe Sensoreinheit (18) eine Energieempfangseinheit (28) zu einer kontaktlosen Energieversorgung aufweist.

## Claims

1. Mobile measuring apparatus, in particular moisture measuring apparatus, having an integrated sensor unit (12) which is configured to capture at least one moisture characteristic value of at least one building material (14), and having a communication unit (16) which is at least configured to receive at least one signal from at least one external sensor unit (18), wherein the at least one signal comprises at least one further moisture characteristic value of the at least one building material (14), **characterized by** a computing unit (20) which is configured to compute the at least one moisture characteristic value with the at least one further moisture characteristic value.

2. Mobile measuring apparatus according to Claim 1, **characterized in that** the communication unit (16) is configured to wirelessly receive the at least one signal.

3. Mobile measuring apparatus according to Claim 1, **characterized in that** the computing unit (20) is configured to determine at least one compensation characteristic variable from the at least one moisture characteristic value and the at least one further moisture characteristic value.

4. Mobile measuring apparatus according to one of the preceding claims, **characterized by** at least one interface (22) which is configured to transmit data to at least one external data processing unit.

5. Mobile measuring apparatus according to one of the preceding claims, **characterized by** an energy transmission unit (24) which is configured to contactlessly supply energy to the at least one external sensor unit (18).

6. Moisture measuring system having at least one mobile measuring apparatus (10) according to one of Claims 1 to 5 and at least one external sensor unit, wherein the external sensor unit (18) has at least one sensor (42, 44) which is configured to capture at least one moisture characteristic value of a building material (14) .

7. Moisture measuring system according to Claim 6, **characterized in that** the at least one external sensor unit (18) is configured to be at least partially embedded in the at least one building material (14).

8. Moisture measuring system according to Claim 6 or 7, **characterized in that** the at least one external sensor unit (18) has a further communication unit (26) which is configured to emit the at least one signal.

9. Moisture measuring system according to one of Claims 6 to 8, **characterized in that** the at least one external sensor unit (18) has an energy reception unit (28) for contactlessly supplying energy.

## Revendications

1. Dispositif de mesure mobile, notamment dispositif de mesure d'humidité, comprenant une unité de détection intégrée (12) qui est conçue pour acquérir au moins une valeur caractéristique d'humidité d'au moins un matériau de construction (14), et comprenant une unité de communication (16) qui est au moins conçue pour recevoir au moins un signal d'au moins une unité de détection externe (18), l'au moins un signal contenant au moins une valeur caractéristique d'humidité supplémentaire de l'au moins un matériau de construction (14), **caractérisé par** une unité de calcul (20) qui est conçue pour compenser l'au moins une valeur caractéristique d'humidité avec l'au moins une valeur caractéristique d'humidité supplémentaire.

2. Dispositif de mesure mobile selon la revendication 1, **caractérisé en ce que** l'unité de communication (16) est conçue pour la réception sans fil de l'au moins un signal.

3. Dispositif de mesure mobile selon la revendication 1, **caractérisé en ce que** l'unité de calcul (20) est conçue pour déterminer au moins une grandeur caractéristique de compensation à partir de l'au moins une valeur caractéristique d'humidité et de l'au moins une valeur caractéristique d'humidité supplémentaire.

4. Dispositif de mesure mobile selon l'une des revendications précédentes, **caractérisé par** au moins une interface (22) qui est conçue pour communiquer des données à au moins une unité de traitement de données externe.

5. Dispositif de mesure mobile selon l'une des revendications précédentes, **caractérisé par** une unité d'émission d'énergie (24) qui est conçue pour une alimentation en énergie sans contact de l'au moins une unité de détection externe (18).

6. Système de mesure d'humidité comprenant au moins un dispositif de mesure mobile (10) selon l'une des revendications 1 à 5 et au moins une unité de détection externe, l'unité de détection externe (18) possédant au moins un capteur (42, 44) qui est conçu pour acquérir au moins une valeur caractéristique d'humidité d'au moins un matériau de construction (14).

7. Système de mesure d'humidité selon la revendication 6, **caractérisé en ce que** l'au moins une unité de détection externe (18) est conçue pour un enrobage au moins partiel dans l'au moins un matériau de construction (14).

8. Système de mesure d'humidité selon la revendication 6 ou 7, **caractérisé en ce que** l'au moins une unité de détection externe (18) possède une unité de communication supplémentaire (26) qui est conçue pour émettre l'au moins un signal.

9. Système de mesure d'humidité selon l'une des revendications 6 à 8, **caractérisé en ce que** l'au moins une unité de détection externe (18) possède une unité de réception d'énergie (28) destinée à une alimentation en énergie sans contact.
